# EUROPEAN PATENT APPLICATION

(11) **EP 1 977 769 A1**
(43) Date of publication of application: **08.10.2008**
(21) Application number: 07007078.4
(22) Date of filing: 04.04.2007
(51) Int. Cl.: A61K 51/04

(54) **Kit for the preparation of a radioactive iodine marker comprising lyophilized meta-iodobenzyl guanidine (MIBG), a method of preparation thereof and a method of radiolabelling said MIBG.**

(71) Applicant: Atomic Energy Council - Institute of Nuclear Energy Research, Taoyuan County (TW)
(72) Inventor: Luo, Tsai-Yueh, Longtan Township Taoyuan County 32546 (TW); Liang, Te-sheng, Longtan Township Taoyuan County 32546 (TW); Shing, Chang-Mau, Longtan Township Taoyuan County 32546 (TW); Farn, Shiou-Shiow, Longtan Township Taoyuan County 32546 (TW)
(74) Representative: Kador & Partner

(57) **Abstract**

The present invention relates to a kit comprising lyophilized meta-iodobenzyl guanidine (MIBG), a method of preparation thereof and a method of radiolabelling said MIBG to obtain a radioactive marker. The Kit comprises the lyophilized MIBG in a first container and sodium acetate buffer in a second container. The method of making said kit comprises the steps of first mixing MIBG, sulfuric acid and ammonium sulfate in pyrogen-free water and freeze-drying said mixture in a first container and sterilizing the sodium acetate buffer in a second container. The method of radiolabelling to obtain a marker comprises the steps of reacting the lyophilized MIBG with radioactive iodine ion and adding the buffer solution. The present invention allows for easy and instant use of the radioactive marker in order to avoid loss of radioactivity.

## Description

### BACKGROUND OF THE INVENTION

### Field of the Invention

The present invention relates to a combination set of MIBG freezing crystal and making method thereof and method of radioactive marker for making, and more particularly, to a combination set of MIBG freezing crystal of blending simply and non- restraint radioactive material. The combination set of MIBG freezing crystal is blending simply. After the blending process, the radioactive iodine marker of Meta-Iodobenzyl Guanidine (MIBG) can be used instantly so the activity of radioactive iodine marker of MIBG is not easily reduced. The radioactive iodine marker of MIBG can be used for medicine radiography, so as to diagnose the diseases.

### Description of the Prior Art

The radioactive iodine marker of Meta-Iodobenzyl Guanidine (MIBG) has the functions of efficient diagnosis and fixed position for original neuron tumors, like malignant schwannoma, carcinoid tumor, neuroblastoma, pheochromocytoma, paraganglioma and thyroid medullary carcinoma, etc. Furthermore, the radioactive iodine marker is respected to diagnose the function of the heart muscles sympathetic nerve in clinic. The radioactive iodine markers have different isotopes so they have different purposes. Like the I-131, its half-life is longer. Also, the I-131 can release β particle and it has a stronger ionization function so it can be used for the diagnosis and even used for the tumor therapy. As for the radioactive iodine markers of I-123, it is a pure γ radioactive source and the energy of theγray is 159 kev so it is suitable for radiography. The used dosage of the radioactive iodine markers of I-123 is less than the used dosage of the radioactive iodine markers of I-131 and the whole image quality of the radioactive iodine markers of I-123 is more ideal than the radioactive iodine markers of I-131. Hence, the radioactive iodine markers of I-123 should be used for the disease diagnosis and the radioactive iodine markers of I-131 should be used for the therapy.

The common methods of the iodine markers can be classified into two types. One type is an oxidation-iodide method and the oxidation-iodide method is suitably used for proteins and the organic molecules having no halogen ions and no other leaving groups. Another type is a substituted iodide method, which is used for the organic molecules having halogen ions and other leaving groups.

When the organic molecule has a little molecular weight and it has the iodine atom or the leaving group, the iodine atom or the leaving group can be substituted by a radioactive iodine ion, so as to form a simple-radioactive iodine marker. This substituted reaction can proceed in solid or liquid status. In the past, people also used Cu(I), the condition of excess reducing agent or the exchange reaction of the isotope iodine to manufacture the radioactive iodine marker of Meta-Iodobenzyl Guanidine of I-123 or I-131. Although high marker efficiency is got, the PH value must be adjusted during the processes of manufacturing radioactive iodine marker. Hence, the operator will suffer higher radiation dosage. Also, in the prior art, the reaction processes are too complex and the radioactive iodine marker must be manufactured in the pharmaceutical factory and then is sent into hospital to use. Furthermore, like I-123, its half-life is only 13.2 hours. The activity of the product is already reduced after adjusting agent and quality control testing. Hence, I-123 of higher activity must join the reaction to satisfy the clinical requirement. Although the I-131 has a longer half-life ( 8 days), the activity of I-131 is great reduced after adjusting agent completely and then sending into the hospital. The hospital wants to use conveniently so the I-131 of lower activity is used for radiography diagnosis of the patient. Nevertheless, γ ray's energy of the I-131 is higher (365 keV) than I-123 and the radiography quality of the I-131 is worse than I-123 and the patient will suffer higher radiation dosage.

Accordingly, prior Cu(I), the condition of the excess reducing agent or the exchange reaction of the isotope iodine is used to manufacture the radioactive iodine marker of Meta-Iodobenzyl Guanidine of I-123 or I-131 that must adjust PH value. Hence, the operator will suffer higher radiation dosage. Like the I-123, its the activity is great reduced after sending into the hospital. Although the I-131 has a longer half-life, the activity of I-131 is great reduced after adjusting agent completely and then sending into the hospital. Hence, the I-131 of lower activity is used for radiography diagnosis of the patient. Though, the I-131 has higher γ ray's energy and the radiography quality of the I-131 is worse than I-123 and the patient will suffer higher radiation dosage.

Accordingly, the inventor want to improve the defects of the prior arts so a combination set of MIBG freezing crystal and making method thereof and method of radioactive marker for making is provided. The combination set of freezing crystal is very simple and easy to use during the adjusting agent processes. The nucleon medicine department or nucleon dispensary of the hospital can adjust agents by itself and the reaction time is just about one hour to get the radioactive iodine marker of Meta-Iodobenzyl Guanidine. Hence, the radioactive iodine marker of Meta-Iodobenzyl Guanidine will not lose its activity easily before using it.

### SUMMARY OF THE INVENTION

The primary objective of the present invention provides a combination set of Meta-Iodobenzyl Guanidine (MIBG) freezing crystal and making method thereof and method of radioactive marker for making. It is very simple and easy to use during the adjusting agent processes and the reaction time is just about one hour. The nucleon medicine department or nucleon dispensary of the hospital can adjust agents by itself according to the clinical requirement.

The next objective of the present invention provides a combination set of Meta-Iodobenzyl Guanidine (MIBG) freezing crystal and making method thereof and making method of radioactive iodine marker. The design of the combination set of freezing crystal is not limited to radioactive material controlled. After adjusting agent processes, the radioactive iodine marker don't lose its activity easily before using it.

The present invention provides a combination set of MIBG freezing crystal and making method thereof and method of radioactive marker for making. The combination set of meta-iodobenzyl guanidine freezing crystal includes a first container having freezing crystal; and a second container having sodium acetate buffer solution. The making method of combination set of meta-iodobenzyl guanidine freezing crystal includes the steps of: mixing (meta-iodobenzyl guanidine)₂ • sulfuric acid and ammonium sulfate into first non-pyrogenic injection water; filtering and then getting meta-iodobenzyl guanidine solution; putting the meta-iodobenzyl guanidine solution in a first container; getting the first container having meta-iodobenzyl guanidine freezing crystal after freezing and drying; adding three water sodium acetate and acetic acid into second injection water; filtering and then getting sodium acetate buffer solution; putting the sodium acetate buffer solution in a second container; and getting the second container having the sodium acetate buffer solution after sterilizing. The method of radioactive marker for making includes the steps of: adding radioactive iodine ion into meta-iodobenzyl guanidine freezing crystal to react; and adding sodium acetate buffer solution and then getting radioactive meta-iodobenzyl guanidine solution.

These and other objectives of the present invention will no doubt become obvious to those of ordinary skill in the art after reading the following detailed description of the preferred embodiment that is illustrated in the various figures and drawings.

### BRIEF DESCRIPTION OF THE DRAWINGS

Fig. 1 is a flow chart of processes of a making method of a combination set of freezing crystal according to the present invention.
Fig. 2 is a flow chart of processes of a making method of a radioactive iodine marker according to the present invention.
Fig. 3 is a radio-thin layer analysis scan graph of the product of radioactive I-123 Meta-Iodobenzyl Guanidine (MIBG) of the preferred embodiment according to the present invention.
Fig. 4 is a high efficiency liquid chromatograph scan graph of the product of radioactive I-123 Meta-Iodobenzyl Guanidine (MIBG) of the preferred embodiment according to the present invention.

### DETAILED DESCRIPTION OF THE PREFERRED EMBODIMENT

The feature of the combination set of meta-iodobenzyl guanidine (MIBG) freezing crystal and making method thereof and method of radioactive marker for making according to present invention is further understood by examiner so the preferred embodiment is illustrated as following.

The combination set of meta-iodobenzyl guanidine (MIBG) freezing crystal according the present invention includes a first container having freezing crystal; and a second container having sodium acetate buffer solution. The meta-iodobenzyl guanidine freezing crystal and the sodium acetate buffer solution are mixed at using time.

The (meta-iodobenzyl guanidine)₂ · sulfuric acid and ammonium sulfate are mixed to form the meta-iodobenzyl guanidine (MIBG) freezing crystal.

The concentration of the sodium acetate buffer solution is 0.1~0.01 M and the preferred concentration of the sodium acetate buffer solution is 0.05 M.

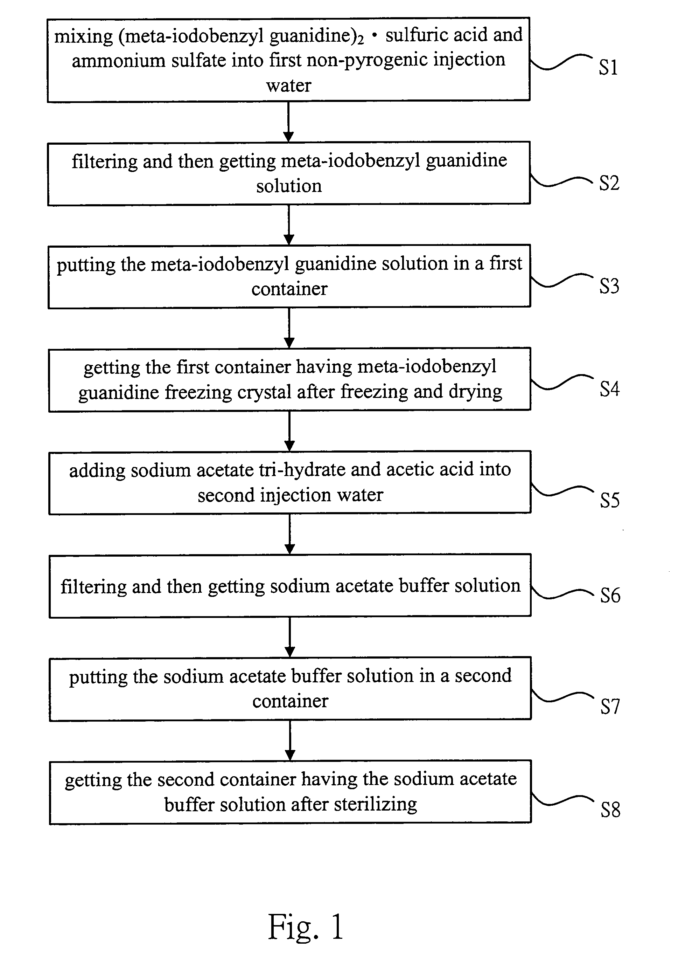

The making method of combination set of meta-iodobenzyl guanidine freezing crystal according the present invention includes the steps of (as show in Fig. 1):
S1 mixing (meta-iodobenzyl guanidine)₂ · sulfuric acid and ammonium sulfate into first non-pyrogenic injection water;
S2 filtering and then getting meta-iodobenzyl guanidine solution;
S3 putting the meta-iodobenzyl guanidine solution in a first container;
S4 getting the first container having meta-iodobenzyl guanidine freezing crystal after freezing and drying;
S5 adding sodium acetate tri-hydrate and acetic acid into second injection water;
S6 filtering and then getting sodium acetate buffer solution;
S7 putting the sodium acetate buffer solution in a second container; and
S8 getting the second container having the sodium acetate buffer solution after sterilizing.

After the step of S4 getting the first container having meta-iodobenzyl guanidine freezing crystal after freezing and drying, further includes a step of sealing the first container.

After the step of S8 getting the second container having the sodium acetate buffer solution after sterilizing, further includes a step of sealing the second container. In the step of S2 filtering and then getting meta-iodobenzyl guanidine solution, a first sterile filter membrane is used. In the step of S6 filtering and then getting sodium acetate buffer solution, a second sterile filter membrane is used. The diameters of the first and second sterile filter membrane are 0.22 µm. In the step of S4 getting the first container having meta-iodobenzyl guanidine freezing crystal after freezing and drying, the time of freezing and drying is 15~30 hours. In the step of S8 getting the second container having the sodium acetate buffer solution after sterilizing, the step of sterilizing is a steam sterilizing step. The temperature of steam sterilizing is 110~130°C and the preferred temperature of the steam sterilizing step is 120°C.

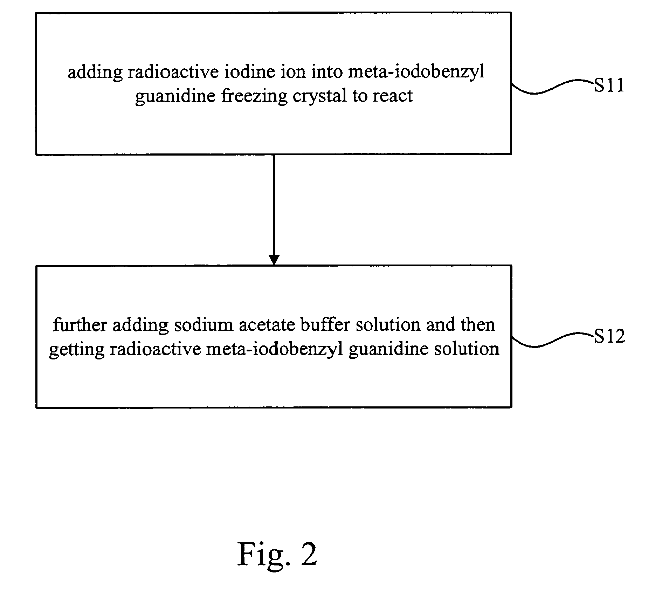

The method of radioactive marker for making according to the present invention includes the steps of (as show in Fig. 2):
S11 adding radioactive iodine ion into meta-iodobenzyl guanidine freezing crystal to react; and
S12 further adding sodium acetate buffer solution and then getting radioactive meta-iodobenzyl guanidine solution.

In the step of S11 adding radioactive iodine ion into meta-iodobenzyl guanidine freezing crystal to react, the reaction temperature is 140~160°C. In the step of S11 adding radioactive iodine ion into meta-iodobenzyl guanidine freezing crystal to react, the preferred reaction temperature is 150 °C. After the step of S11 adding radioactive iodine ion into meta-iodobenzyl guanidine freezing crystal to react, further includes a step of cooling to room temperature. In the step of S11 adding radioactive iodine ion into meta-iodobenzyl guanidine freezing crystal to react, the reaction time is 60~90 minutes. In the step of S11 adding radioactive iodine ion into meta-iodobenzyl guanidine freezing crystal to react, the radioactive sodium iodide dissociates the radioactive iodine ion, which is selected from the group consisting of radioactive 1-123 and radioactive 1-131. Iodine of the meta-iodobenzyl guanidine freezing crystal and the radioactive iodine ion are exchanged by substituted reaction and then getting the radioactive meta-iodobenzyl guanidine. In the step of S12 further adding sodium acetate buffer solution and then getting radioactive meta-iodobenzyl guanidine solution, the radioactive meta-iodobenzyl guanidine solution is radioactive iodine marker solution.

The preferred embodiment of the combination set of meta-iodobenzyl guanidine (MIBG) freezing crystal according the present invention is show as following:
First, ammonium sulfate is added into 1 mg (meta-iodobenzyl guanidine)₂ · sulfuric acid and mixing thereof into non-pyrogenic injection water to form the solution. Then, the solution is filtered by the sterile filter membrane (its diameter is 0.22 µm) to get the meta-iodobenzyl guanidine solution and then the meta-iodobenzyl guanidine solution is separated into a plurality of small bottles. The small bottles having the meta-iodobenzyl guanidine solution are put in freezing and drying mechanism about 15 hours after the small bottles are taken out and sealed to get the small bottles having the meta-iodobenzyl guanidine (MIBG) freezing crystal.

The sodium acetate tri-hydrate and acetic acid are added in the injection water and dissolved to get the sodium acetate buffer solution and then the sodium acetate buffer solution is filtered by the sterile filter membrane (its diameter is 0.22 µm). The sodium acetate buffer solution is separated into a plurality of small bottles and then the small bottles having the sodium acetate buffer solution, which are sterilized at 120°C to get a plurality of small bottles having sodium acetate buffer solution (0.05 M).

The preferred embodiment of the method of radioactive marker for making according to the present invention is described as following:

The solution of the radioactive I-123 or I-131 is added in the small bottle having the meta-iodobenzyl guanidine (MIBG) freezing crystal and reacting with thereof to form a mixing solution at 150°C in 60 minutes. After the mixing solution is cooled to the room temperature, the sodium acetate buffer solution is added into the mixing solution to get the product of radioactive iodine marker of I-123 or I-131 meta-iodobenzyl guanidine (MIBG).

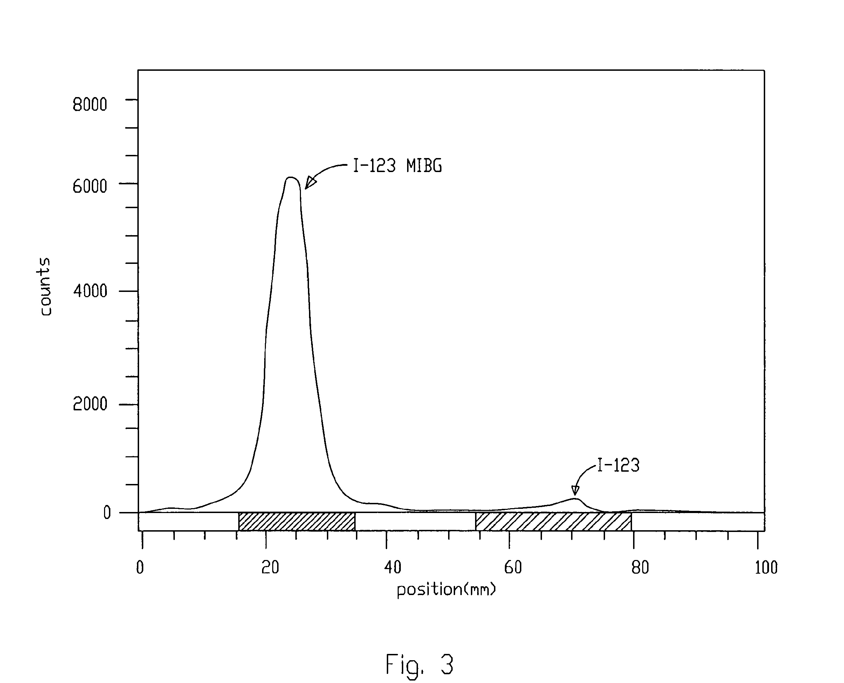

The pure degree of the product of radioactive iodine marker of 1-123 meta-iodobenzyl guanidine (MIBG) is measured by the analysis of pure degree of the radioactive chemistry. In the analysis of pure degree of the radioactive chemistry, the fixed phase sheet is a silica-gel coated aluminum plate. The compositions of the spreading agent are C₂H₅OH and NH₄OH (C₂H₅OH/ NH₄OH(10%)=3/1). After the product of radioactive iodine marker of I-123 meta-iodobenzyl guanidine (MIBG) is spread on the sheet, the sheet having the product of radioactive iodine marker of I-123 meta-iodobenzyl guanidine is scanned by the radio-thin layer analysis instrument (as shown in Fig. 3 and table 1) and then the data of Table 1 is got.

**Table 1 The result of the product of radioactive iodine marker of I-123 meta-iodobenzyl guanidine (MIBG) scanned by radio-thin layer analysis instrument**

| Reg | Start (mm) | Stop (mm) | Centroid (mm) | RF | Region counts | Total CPM | % of Total | % of ROI |
|---|---|---|---|---|---|---|---|---|
| Rgn 1 | 15.5 | 34.5 | 24.1 | 0.166 | 56114.0 | 87.94 | 87.94 | 95.41 |
| Rgn 2 | 54.4 | 79.6 | 66.8 | 0.941 | 2700.0 | 4.23 | 4.23 | 4.59 |
| 1 Peak | | | | | 58814.0 | 58814.0 | 92.17 | 100.0 |

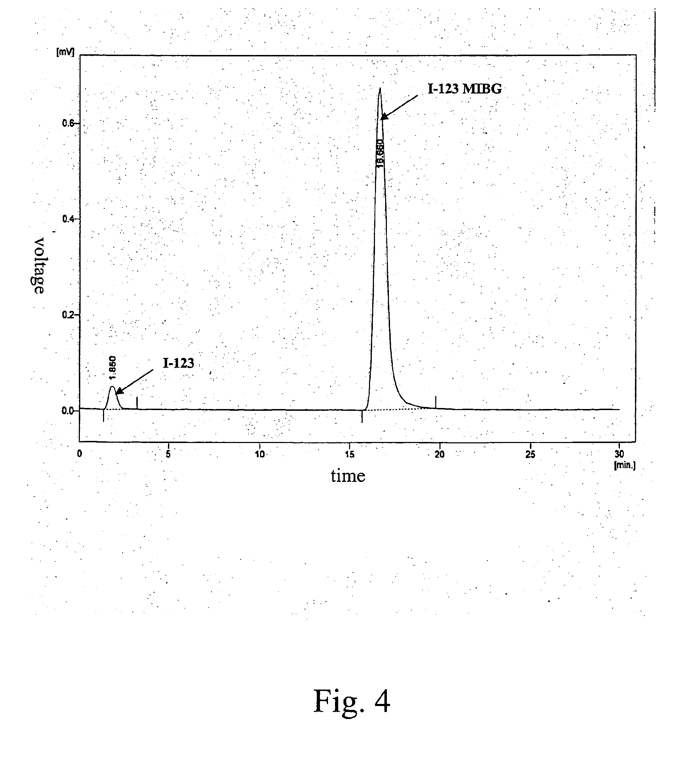

The RF value of the I-123(or I-131) MIBG is about 0.16 on the sheet and the RF value of the I-123 sodium iodide is between 0.9 and 1.0 so the product's radioactive chemistry pure degree is up to 95.41 % (as show in Fig. 3). The radioactive chemistry pure degree of the same product is 95.3% that is measured by a high efficiency liquid chromatograph instrument (as show in Fig. 4 and table 2). Accordingly, the yield of radioactive iodine marker of I-123 meta-iodobenzyl guanidine (MIBG) is very high.

**Table 2 The result of the product of radioactive iodine marker of I-123 meta-iodobenzyl guanidine (MIBG) scanned by high efficiency liquid chromatograph instrument**

| Reten. time | Area [mV.s] | Height [mV] | Area [%] | Height [%] | W05 [min] |
|---|---|---|---|---|---|
| 1.850 | 1593.934 | 48.476 | 4.7 | 6.7 | 0.53 |
| 16.660 | 32004.766 | 671.414 | 95.3 | 93.3 | 0.74 |
| total | 33598.700 | 719.890 | 100.0 | 100.0 | |

The present invention relates to a combination set of MIBG freezing crystal. The meta-iodobenzyl guanidine can react by *I-I substitution in the condition of solid ammonium sulfate. The composition of meta-iodobenzyl guanidine and ammonium sulfate has a proper ratio to form the MIBG freezing crystal and a bottle having the buffer solution that is the combination set of MIBG freezing crystal at present invention. The advantages of the combination set of MIBG freezing crystal are as following:
(1) Adjusting agent and using are very easy. Also, the nucleon medicine department or nucleon dispensary of the hospital can adjust agents by itself and the reaction time is just about one hour to get the radioactive iodine marker of Meta-Iodobenzyl Guanidine. Furthermore, the money and therapy time are not wasted because the product don't import from abroad.
(2) The design of the combination set of MIBG freezing crystal of the present invention is not limited by radioactive material controlled and the activity is not reduced by the time. The hospital can purchase and save the product of the present invention at any time and the product can be delivered to foreign hospitals.
The hospital or dispensary can buy the radioactive liquid of the I-123 or I-131 sodium iodine by itself to match the combination set of MIBG freezing crystal of the present invention. The radioactive liquid of the I-123 or I-131 sodium iodine is added into the MIBG freezing crystal and then heat to dryness at 150°C and continued one hour. After the heat process is finished, the buffer solution of the combination set of MIBG freezing crystal is added and then the product of radioactive iodine marker of 1-123 meta-iodobenzyl guanidine (MIBG) is got. Due to adjusting agent at using time, the product of radioactive iodine marker of I-123 or I-131 meta-iodobenzyl guanidine (MIBG) don't lose its activity before using.

Through many marker experiments, the present invention is proved to operate easily. The marker efficiency of radioactive iodine marker of 1-123 meta-iodobenzyl guanidine is upper to 95% and the PH value of the product is 4~6. The occupied ratio of the non-reaction's I-123 or I-131 is very low and the product fits in with the standards of USP and BP pharmacopoeia.

Those skilled in the art will readily observe that numerous modifications and alterations of the device may be made while retaining the teachings of the invention. Accordingly, the above disclosure should be construed as limited only by the metes and bounds of the appended claims.

## Claims

1. A combination set of meta-iodobenzyl guanidine freezing crystal comprising:
a first container having a freezing crystal; and
a second container having a sodium acetate buffer solution;
wherein mixing the meta-iodobenzyl guanidine freezing crystal and the sodium acetate buffer solution at using time.

2. The combination set of meta-iodobenzyl guanidine freezing crystal in claim 1 wherein a concentration of the sodium acetate buffer solution is 0.1~0.01 M.

3. The combination set of meta-iodobenzyl guanidine freezing crystal in claim 1 wherein a preferred concentration of the sodium acetate buffer solution is 0.05 M.

4. A method for making a combination set of meta-iodobenzyl guanidine freezing crystal comprising the steps of:
mixing (meta-iodobenzyl guanidine)₂ · sulfuric acid and ammonium sulfate into a first non-pyrogenic injection water;
filtering and then getting meta-iodobenzyl guanidine solution;
putting the meta-iodobenzyl guanidine solution in a first container;
getting the first container having meta-iodobenzyl guanidine freezing crystal after freezing and drying;
adding sodium acetate tri-hydrate and acetic acid into a second injection water;
filtering and then getting sodium acetate buffer solution;
putting the sodium acetate buffer solution in a second container; and
getting the second container having the sodium acetate buffer solution after sterilizing.

5. The method for making a combination set of meta-iodobenzyl guanidine freezing crystal in claim 4 further comprising a step of sealing the first container after the step of getting the first container having meta-iodobenzyl guanidine freezing crystal after freezing and drying.

6. The method for making a combination set of meta-iodobenzyl guanidine freezing crystal in claim 4 further comprising a step of sealing the second container after the step of getting the second container having the sodium acetate buffer solution after sterilizing.

7. The method for making a combination set of meta-iodobenzyl guanidine freezing crystal in claim 4 wherein the step of getting the first container having meta-iodobenzyl guanidine freezing crystal after freezing and drying, a time of freezing and drying is 15~30 hours.

8. The method for making a combination set of meta-iodobenzyl guanidine freezing crystal in claim 4 wherein the step of getting the second container having the sodium acetate buffer solution after sterilizing, the step of sterilizing is a steam sterilizing step.

9. The method for making a combination set of meta-iodobenzyl guanidine freezing crystal in claim 8 wherein the temperature of the steam sterilizing step is 110~130°C.

10. The method for making a combination set of meta-iodobenzyl guanidine freezing crystal in claim 8 wherein the preferred temperature of the steam sterilizing step is 120°C.

11. A method for making a radioactive marker comprising the steps of:
adding radioactive iodine ion into meta-iodobenzyl guanidine freezing crystal to react; and
adding sodium acetate buffer solution and then getting radioactive meta-iodobenzyl guanidine solution.

12. The method for making a radioactive marker in claim 11 wherein the reaction temperature of the step of adding the radioactive iodine ion into the meta-iodobenzyl guanidine freezing crystal to react is 140~160°C.

13. The method for making a radioactive marker in claim 11 wherein the preferred temperature of the step of adding the radioactive iodine ion into the meta-iodobenzyl guanidine freezing crystal to react is 150 °C.

14. The method for making a radioactive marker in claim 13 further comprising a step of cooling to room temperature after adding the radioactive iodine ion into the meta-iodobenzyl guanidine freezing crystal to react.

15. The method for making a radioactive marker in claim 11 wherein the reaction time of the step of adding the radioactive iodine ion into the meta-iodobenzyl guanidine freezing crystal to react is 60~90 minutes.

16. The method for making a radioactive marker in claim 11 wherein the step of adding the radioactive iodine ion into the meta-iodobenzyl guanidine freezing crystal to react, the radioactive iodine ion is from dissociated radioactive sodium iodide.

17. The method for making a radioactive marker in claim 16 wherein the radioactive iodine ion is selected from the group consisting of radioactive I-123 and radioactive I-131.

18. The method for making a radioactive marker in claim 11 wherein the step of adding the radioactive iodine ion into the meta-iodobenzyl guanidine freezing crystal to react, iodine of the meta-iodobenzyl guanidine freezing crystal and the radioactive iodine ion are exchanged by substituted reaction and then getting the radioactive meta-iodobenzyl guanidine.

19. The method for making a radioactive marker in claim 11 wherein the step of adding sodium acetate buffer solution and then getting radioactive meta-iodobenzyl guanidine solution, the radioactive meta-iodobenzyl guanidine solution is radioactive iodine marker solution.
